Europäisches Patentamt

European Patent Office

Office européen des brevets

(19)

(11) Publication number: **0 250 879 B1**

# EUROPEAN PATENT SPECIFICATION

(12)

(45) Date of publication of patent specification:
09.01.91 Bulletin 91/02

(51) Int. Cl.⁵: **C07C 2/00**

(21) Application number: 87107769.9

(22) Date of filing: 27.05.87

(54) **Direct catalytic alkylation of mononuclear aromatics with lower alkanes.**

(30) Priority: 27.06.86 US 879786

(43) Date of publication of application:
07.01.88 Bulletin 88/01

(45) Publication of the grant of the patent:
09.01.91 Bulletin 91/02

(84) Designated Contracting States:
BE DE FR GB IT NL

(56) References cited:
DE-A- 2 817 577
FR-A- 2 021 410
US-A- 4 085 156
US-A- 4 524 230

(73) Proprietor: **MOBIL OIL CORPORATION**
**3224 Gallows Road**
**Fairfax Virginia 22037-0001 (US)**

(72) Inventor: **LaPierre, Rene Bernard**
**43 Arrowhead Court**
**Medford New Jersey 08055 (US)**
Inventor: **Morrison, Roger Allan**
**57 Jupiter Court**
**Beptford New Jersey 08096 (US)**

(74) Representative: **Kador & Partner**
**Corneliusstrasse 15**
**D-8000 München 5 (DE)**

## Description

This invention relates to a process for converting an aromatic compound, such as benzene, to alkyl aromatic products by direct alkylation with a $C_2$-$C_4$ alkane.

In the past, the alkylation of aromatic hydrocarbons with non-heteroatom-containing hydrocarbons was achieved by two methods. The first method reacts an aromatic hydrocarbon with an olefin ; such as ethylene, propylene, etc. In the second method aromatics are indirectly alkylated with an alkane.

Alkylation of aromatic hydrocarbons with an olefin in the presence of a crystalline metallic aluminosilicate having uniform large pore openings of about 6 to 15 Angstrom units is described in U.S. Pat. No. 2,904,607 (Lavaud). U.S. Pat. No. 3,251,897 (Wise) describes alkylation of aromatic hydrocarbons in the presence of X or Y-type crystalline aluminosilicate zeolites, specifically such large pore zeolites wherein the cation is rare earth and/or hydrogen. U.S. Pat. Nos. 3,751,504 (Keown, et al.) and 3,751,506 (Burress) describe vapor phase alkylation of aromatic hydrocarbons with olefins, e.g. benzene with ethylene, in the presence of a ZSM-5 type medium pore zeolite catalyst. U.S. Pat. No. 3,755,483 (Burress) describes alkylation of aromatic hydrocarbons with olefins in the presence of a crystalline aluminosilicate zeolite material. U.S. Pat. Nos. 4,086,287 (Kaeding, et al.) and 4,117,024 (Kaeding) describe alkylation of monoalkyl benzenes with ethylene in the presence of a crystalline aluminosilicate zeolite material. U.S. Pat. No. 4,375,573 (Young) describes alkylation of mono-substituted aromatic compounds with olefins in the presence of a crystalline aluminosilicate zeolite material.

In the second method, paraffinic hydrocarbons are indirect alkylating agents. U.S. Pat. Nos. 4,085,156 (Frilette, et al.) and 4,157,950 (Frilette, et al.) describe alkylation of aromatic hydrocarbons with materials derived from paraffins or cycloparaffins over a crystalline aluminosilicate zeolite characterized by a silica-to-alumina ratio of at least 10. These paraffins consist essentially of $C_6^+$ hydrocarbons which can contain significant amounts of pentane, but $C_2$-$C_4$ hydrocarbons are not present in significant amounts.

U.S. Pat. No. 4,524,230 (Haensel) describes a process for preparing alkylaromatic compounds by alkylating an aromatic material with paraffinic hydrocarbons, such as ethane, propane, n-butane, iso-butane, etc., over a catalyst having no acid activity. The paraffin material is catalytically decomposed in the presence of the nonacid-acting catalyst to give an olefin fragment which is "scavenged" by the aromatic material. The catalyst is at least one metal of Group VIII of the Periodic Table on a non-acidic solid support.

In the prior art methods, the reactive alkylating species is usually an olefinic material, either added as part of the feed, or generated in situ from paraffins by cracking or dehydrogenation over a metal-containing catalyst. The cracking produces an olefin and a paraffin fragment, thereby limiting alkylation selectivity to about 50%.

There is a need for a simple one-step route to the formation of alkylaromatic compounds directly from aromatics and paraffins.

A one-step process has been discovered for the direct alkylation of aromatic hydrocarbon materials with lower molecular weight paraffins. Hydrogen is not required in the process, eliminating costly hydrogen recirculation.

Accordingly, the present invention provides a process for making alkyl aromatic hydrocarbons characterized by contacting a reaction mixture substantially free of hydrogen and comprising a mononuclear aromatic compound and $C_2$-$C_4$ alkane in a weight ratio of 10 :1 to 1 :10 at 315°C to 480°C and a pressure of 100 to 10,000 kPa with a catalyst comprising an acidic medium pore metallosilicate zeolite.

An aromatic feedstock containing predominantly benzene, toluene or mixtures thereof is co-fed to a catalytic fixed bed reactor in combination with a stream of a low molecular weight $C_2$-$C_4$ paraffin, such as ethane, propane, isobutane, n-butane or mixtures thereof. The weight hourly space velocity (WHSV) of the combined feeds is 0.1-10, based on active catalyst, and the temperature and pressure conditions are generally mild as compared to cracking conditions. Preferred temperatures are 315-480°C (600-900°F), and preferred pressures are 100 to 10,000 kPa (0-1500 psig).

As the combined reactants pass through the fixed bed of alkylation catalyst the low molecular weight paraffins are chemically modified to a selective alkylating agent. In the presence of an alkylatable mononuclear moiety, such as benzene or toluene, the paraffin combines freely to produce aromatic materials having saturated lower aliphatic side-chains. Examples of such products are $C_7$-$C_{10}$ methyl ethyl benzenes, propyl benzenes, and some naphthalenes. If toluene is the feed similar products are formed.

The alkylation of the aromatic feed is conducted in the absence of hydrogen, as this is detrimental to the reaction. In the presence of a significant amount of hydrogen, side reactions, such as hydrocracking, are possible. The hydrogen content of the reaction mixture preferably is less than five moles per mole of hydrocarbon, and most preferably less than one mole per mole of hydrocarbon reactants.

The reaction proceeds efficiently without olefins. The feed can be substantially free of added olefin,

2

although some olefin impurity is present in commercial feedstocks. In a preferred embodiment, the reaction mixture consists essentially of at least one benzenoid compound and propane in a weight ratio of 10 :1 to 1 :10. Alkylation selectivity is at least 50%.

Products generated with a high selectivity during the reaction are the alkylated forms of mononuclear aromatic hydrocarbons. It is believed that the reaction proceeds through a series of dehydrogenation, alkylation, isomerization and disproportionation steps. Although generally of less commercial interest, the products of reactive intermediates would also react with condensed rings, such as naphthalene or methyl naphthalene.

When butane is part of the feed, either the normal or isoform or mixtures can be used, as isomerization of the butane occurs under the reaction conditions.

Naphthalenes may form during the reaction (see Examples) because of hydrogen transfer and disproportionation of reactive alkylated aromatics.

Catalysts employed in the alkylation reactor are porous siliceous acid zeolite materials. Preferred zeolites are ZSM-5, ZSM-11, ZSM-22, ZSM-23, ZSM-35, ZSM-38, ZSM-45, ZSM-48, and/or ZSM-50 and mixtures thereof. Most preferred is HZSM-5.

The preferred crystalline aluminosilicate zeolites have a silica to alumina molar ratio of at least 12, a constraint index (C.I.) of 1 to 12 and acid cracking activity of about 50-1000. Preferred C.I. 1-12 zeolites are ZSM-5, ZSM-11, ZSM-22, ZSM-23, ZSM-35 and ZSM-38. ZSM-5 is disclosed in U.S. 3,702,886 ; ZSM-11 is disclosed in U.S. Pat. No. 3,709,979 . Also, see U.S. 3,832,449 for ZSM-12 ; U.S. 4,076,842 for ZSM-23 ; U.S. 4,016,245 for ZSM-35 ; U.S. 4,046,859 for ZSM-38. H-ZSM-5 zeolite (silica : alumina ratio = 40 :1) with alumina binder in the form of cylindrical extrudates of 1-5mm works very well. Other zeolites which may be used include a variety of medium pore (5 to 8 A) siliceous materials ; such as zeolite beta, gallo-silicates, ferrosilicates, and/or aluminosilicates disclosed in U.S. 4,414,413 and 4,417,088 and 3,308,069 (Reissue 28,341). Preferably the zeolites have a crystal size of 0.02 to 2.0 microns.

The preferred zeolites have an effective pore size of 5 to 8 Angstroms, such as to freely sorb normal hexane. The zeolite should provide constrained access to larger molecules. A convenient measure of access is the Constraint Index. Zeolites which provide a highly restricted access to and egress from its internal structure have a high Constraint Index, and usually have pores of small size, e.g., less than 5 Angstroms. Zeolites which provide relatively free access to the internal zeolite structure have a low Constraint Index, and usually pores of large size, e.g. greater than 8 Angstroms. Constraint Index is described in U.S. Pat. No. 4,016,218 (Haag, et al.).

Constraint Index (CI) values for some typical materials are :

|  | CI | (at test temperature) |
|---|---|---|
| ZSM-4 | 0.5 | (316°C) |
| ZSM-5 | 6-8.3 | (371°C – 316°C) |
| ZSM-11 | 5-8.7 | (371°C – 316°C) |
| ZSM-12 | 2.3 | (316°C) |
| ZSM-20 | 0.5 | (371°C) |
| ZSM-22 | 7.3 | (427°C) |
| ZSM-23 | 9.1 | (427°C) |
| ZSM-34 | 50 | (371°C) |
| ZSM-35 | 4.5 | (454°C) |
| ZSM-38 | 2 | (510°C) |
| ZSM-48 | 3.5 | (538°C) |
| ZSM-50 | 2.1 | (427°C) |
| TMA Offretite | 3.7 | (316°C) |
| TEA Mordenite | 0.4 | (316°C) |
| Clinoptilolite | 3.4 | (510°C) |
| Mordenite | 0.5 | (316°C) |
| REY | 0.4 | (316°C) |
| Amorphous Silica-alumina | 0.6 | (538°C) |
| Dealuminized Y | 0.5 | (510°C) |
| Erionite | 38 | (316°C) |
| Zeolite Beta | 0.6-2.0 | (316°C-399°C) |

A given zeolite can be tested under different conditions and exhibit different Constraint Indices. C.I. may vary with severity (conversion) and the presence or absence of binders. Variables, such as crystal size of the zeolite, the presence of occluded contaminants, etc., may affect the C.I.. This explains the range of C.I. for ZSM-5, ZSM-11 and Beta.

The crystalline metallosilicate zeolite catalyst employed in the invention is in the acid form. However, the acid sites on the catalyst can be partially ion-exchanged with metals. Suitable metal cations for exchange include Groups I through VIII of the Periodic Table, including, by way of example, nickel, copper, zinc, palladium, calcium or rare earth metals. Metals may be added to the zeolite by ion exchange or other techniques such as impregnation.

## EXAMPLE 1

Benzene and propane were fed continuously to a fixed bed isothermal tubular catalytic reactor. The bed is packed with H-ZSM-5 having a silica to alumina ratio of 40, and crystal size of .02 to .05 microns. The acid cracking (alpha) value of the catalyst is 513.

The reaction was conducted at 385-399°C (725-750°F) a pressure of 6,184 kPa (900 psig) in the absence of hydrogen. The weight hourly space velocity (WHSV) of the feed was 2.1-2.2. The test ran for 8 days.

Products are analyzed at regular intervals to determine conversion and yields. Results are shown in Table I.

### Table 1

| Charge (wt.%) | | | | | |
|---|---|---|---|---|---|
| Propane | 35 | 36 | 34 | 36 | 36 |
| Benzene | 65 | 64 | 66 | 64 | 64 |
| Time on Stream (hrs.) | 49 | 121 | 145 | 173 | 193 |
| Temperature (C°) | 385 | 385 | 399 | 399 | 399 |
| Pressure (Kpa) | 6183 | 6183 | 6183 | 6183 | 6183 |
| | | | | | |
| Product, wt % | | | | | |
| $C_1$ | 2.07 | 2.07 | 3.21 | 2.75 | 2.80 |
| $C_2$ | 1.45 | 1.43 | 3.07 | 2.79 | 2.59 |
| Iso-$C_4$ | 0.73 | 0.69 | 1.19 | 1.14 | 1.15 |
| N-$C_4$ | 1.04 | 1.06 | 1.46 | 1.48 | 1.54 |
| Iso-$C_5$ | 0.08 | 0.08 | 0.15 | 0.14 | 0.14 |
| N-$C_5$ | 0.06 | 0.06 | 0.10 | 0.10 | 0.10 |
| 2-M-$C_5$ | 0.01 | 0.01 | 0.01 | 0.01 | 0.01 |
| 3-M-$C_5$ | 0.00 | 0.00 | 0.01 | 0.01 | 0.01 |
| N-$C_6$ | 0.01 | 0.01 | 0.01 | 0.01 | 0.01 |
| $C_{13+}$ | 0.30 | 0.24 | 0.58 | 0.54 | 0.48 |
| benzene | 36.19 | 44.86 | 43.86 | 33.86 | 35.39 |
| propane | 19.33 | 23.75 | 26.78 | 18.03 | 18.71 |
| toluene | 9.92 | 8.48 | 18.71 | 17.50 | 16.75 |
| $C_8$ A | 11.86 | 11.24 | 13.24 | 13.00 | 12.78 |
| $C_9$ A | 2.27 | 2.55 | 4.31 | 4.10 | 3.92 |
| $C_{10}$ mononuclear A | 1.56 | 1.11 | 1.02 | 1.70 | 1.62 |
| $C_{11}$-$C_{12}$ mononuclear A | 0.25 | 0.22 | 0.44 | 0.40 | 0.36 |
| Naphthalene | 0.23 | 0.19 | 0.32 | 0.30 | 0.28 |
| | | | | | |
| Total Wt.% Conversion | 31.39 | 29.36 | 48.52 | 45.90 | 44.48 |
| Wt.% $C_3H_8$ Conversion | 32.39 | 25.53 | 46.80 | 48.46 | 46.50 |
| Wt.% $C_6H_6$ Conversion | 30.85 | 31.51 | 49.39 | 44.44 | 43.34 |
| | | | | | |
| Wt.% Selectivity | | | | | |
| $C_1$-$C_2$ | 11.21 | 11.92 | 12.94 | 12.07 | 12.13 |
| $C_4$ | 5.64 | 5.96 | 5.46 | 5.71 | 6.05 |
| $C_5$-$C_6$ | 0.51 | 0.55 | 0.57 | 0.59 | 0.61 |
| $C_{7+}$ A | 82.63 | 81.54 | 81.00 | 81.61 | 81.23 |

In Table 1, the benzene and propane are present in large amounts because the feed is all benzene and propane. A is aromatic, i.e., $C_7^+$ A represents $C_7^+$ aromatics.

## EXAMPLE 2

A mixture of benzene and propane was fed continuously to a fixed bed isothermal tubular catalytic reactor. The catalyst was H-ZSM-12, silica-to-alumina ratio of 76, and alpha value of 160.

The reaction conditions are given in Table 2. The weight hourly space velocity (WHSV) of the feed is 3.1-3.3.

### Table 2

| Charge (wt.%) | | |
|---|---|---|
| Propane | 50.6 | 53.1 |
| Benzene | 49.3 | 46.7 |
| Time on Stream (hrs.) | 4.8 | 2.3 |
| Temperature (°C) | 453 | 481 |
| Pressure (Kpa) | 5497 | 5497 |
| | | |
| **Product, wt %** | | |
| $C_1$ | 0.43 | 1.12 |
| $C_2$ | 0.28 | 0.86 |
| Iso-$C_4$ | 0.64 | 1.23 |
| N-$C_4$ | 0.90 | 1.80 |
| Iso-$C_5$ | 0.08 | 0.16 |
| N-$C_5$ | 0.07 | · 0.14 |
| 2-M-$C_5$ | 0.03 | 0.03 |
| 3-M-$C_5$ | 0.01 | 0.02 |
| N-$C_6$ | 0.03 | 0.03 |
| *$C_{13+}$ | 0.15 | 0.44 |
| benzene | 35.26 | 27.67 |
| propane | 47.16 | 42.02 |
| toluene | 7.03 | 12.74 |
| $C_8$ A | 4.03 | 5.57 |
| $C_9$ A | 1.8 | 2.08 |
| $C_{10}$ mononuclear A | 0.54 | 0.87 |
| $C_{11}$-$C_{12}$ mononuclear A | 0.07 | 0.06 |
| Naphthalene | 0.57 | 1.08 |
| **M-Naphthalenes | 0.90 | 2.03 |
| | | |
| **Total Wt.% Conversion** | 17.58 | 30.30 |
| **Wt.% $C_3H_8$ Conversion** | 6.72 | 20.88 |
| **Wt.% $C_6H_6$ Conversion** | 28.46 | 40.79 |
| **Wt.% Selectivity** | | |
| $C_1$-$C_2$ | 4.04 | 6.53 |
| $C_4$ | 8.76 | 10.00 |
| $C_5$-$C_6$ | 1.25 | 1.32 |
| $C_7$+ aromatics | 85.84 | 82.08 |

*Approximate Analysis

## EXAMPLE 3

A mixture of benzene and propane was fed to a fixed bed isothermal tubular catalytic reactor containing H-ZSM-23, silica-to-alumina ratio of 100, and alpha of 47.

The reaction conditions are give in Table 3. The WHSV is 3-3.1 and no hydrogen is present.

EP 0 250 879 B1

## Table 3

| Charge (wt.%) | | |
|---|---|---|
| Propane | 50.3 | 50.8 |
| Benzene | 49.6 | 49 |
| Time on Stream (hrs.) | 5.5 | 28.8 |
| Temperature ($^{\circ}$C) | 455 | 482 |
| Pressure (Kpa) | 5497 | 5497 |
| | | |
| **Product, wt %** | | |
| $C_1$ | 2.75 | 4.16 |
| $C_2$ | 1.41 | 2.55 |
| Iso-$C_4$ | 0.35 | 0.66 |
| N-$C_4$ | 0.60 | 1.22 |
| Iso-$C_5$ | 0.06 | 0.13 |
| N-$C_5$ | 0.10 | 0.16 |
| 2-M-$C_5$ | 0.03 | 0.04 |
| 3-M-$C_5$ | 0.01 | 0.02 |
| N-$C_6$ | 0.03 | 0.04 |
| *$C_{13+}$ | 0.01 | 0.04 |
| benzene | 36.08 | 32.07 |
| propane | 38.49 | 32.54 |
| toluene | 0.80 | 2.41 |
| $C_8$ A | 13.42 | 15.76 |
| $C_9$ A | 3.16 | 3.56 |
| $C_{10}$ mononuclear A | 1.92 | 2.90 |
| $C_{11}$-$C_{12}$ mononuclear A | 0.27 | 0.28 |
| Naphthalene | 0.05 | 0.15 |
| *M-Naphthalenes | 0.4 | 0.93 |
| | | |
| **Total Wt.% Conversion** | 25.4 | 35.23 |
| **Wt.% $C_3H_8$ Conversion** | 23.42 | 35.97 |
| **Wt.% $C_6H_6$ Conversion** | 27.24 | 34.58 |
| | | |
| **Wt.% Selectivity** | | |
| $C_1$-$C_2$ | 16.38 | 19.05 |
| $C_4$ | 3.78 | 3.66 |
| $C_5$-$C_6$ | 1.10 | 1.36 |
| $C_{7+}$ A | 78.86 | 73.89 |

*Approximate Analysis

## EXAMPLE 4

Benzene and propane were fed to a fixed bed isothermal tubular catalytic reactor containing H-ZSM-11, silica-to-alumina ratio 70 and alpha of 102.

The reaction conditions are given in Table 4. The WHSV is 2.8-3.2. No $H_2$ is added.

6

## Table 4

| Charge (wt.%) | | |
|---|---|---|
| Propane | 49.6 | 46 |
| Benzene | 50.2 | 53.9 |
| Time on Stream (hrs.) | 22.8 | 46.8 |
| Temperature (°C) | 427 | 454 |
| Pressure (Kpa) | 5497 | 5497 |

| Product, wt % | | |
|---|---|---|
| $C_1$ | .93 | 2.66 |
| $C_2$ | .33 | 2.5 |
| Iso-$C_4$ | .34 | 1.46 |
| N-$C_4$ | .5 | 2.07 |
| Iso-$C_5$ | .03 | .17 |
| N-$C_5$ | .04 | .15 |
| 2-M-$C_5$ | .01 | .02 |
| 3-M-$C_5$ | .01 | .01 |
| N-$C_6$ | .01 | .02 |
| *$C_{13+}$ | 0 | .02 |
| benzene | 44.06 | 31.52 |
| propane | 42.07 | 27.48 |
| toluene | 2.33 | 14.59 |
| $C_8$ A | 7.04 | 10.34 |
| $C_9$ A | 1.41 | 3.43 |
| $C_{10}$ mononuclear A | .48 | 1.74 |
| $C_{11}$-$C_{12}$ mononuclear A | .03 | .23 |
| Naphthalene | .15 | .4 |
| *M-Naphthalenes | .22 | 1.15 |

| | | |
|---|---|---|
| Total Wt.% Conversion | 13.87 | 41 |
| Wt.% $C_3H_8$ Conversion | 15.23 | 40.26 |
| Wt.% $C_6H_6$ Conversion | 12.25 | 41.48 |

| Wt.% Selectivity | | |
|---|---|---|
| $C_1$-$C_2$ | 9.08 | 12.59 |
| $C_4$ | 6.06 | 8.61 |
| $C_5$-$C_6$ | .72 | .93 |
| $C_{7+}$ A | 84.07 | 77.8 |

*Approximate Analysis

## EXAMPLE 5

Benzene and propane (no $H_2$) were fed continuously to a fixed bed isothermal tubular catalytic reactor. The catalyst is Zeolite Beta, with silica-to-alumina ratio of 40. The reaction conditions are given in Table 5. The WHSV is 2.8-3.3. No $H_2$ was added.

## Table 5

| Charge (wt.%) | | | |
|---|---|---|---|
| Propane | 52.39 | 54.74 | 51.69 |
| Benzene | 47.45 | 45.08 | 48.16 |
| Time on Stream (hrs.) | 5 | 5.3 | 5 |
| Temperature (°C) | 398 | 453 | 468 |
| Pressure (Kpa) | 5497 | 5497 | 5497 |

| Product, wt % | | | |
|---|---|---|---|
| $C_1$ | 0 | 0 | .25 |
| $C_2 0$ | 0 | 0 | .24 |
| Iso-$C_4$ | .45 | .57 | .70 |
| N-$C_4$ | .23 | .5 | .6 |
| Iso-$C_5$ | .06 | .08 | .09 |
| N-$C_5$ | .03 | .05 | .06 |
| 2-M-$C_5$ | .02 | .02 | .02 |
| 3-M-$C_5$ | .01 | .01 | .01 |
| N-$C_6$ | .01 | .02 | .02 |
| $C_{13+}$ | .12 | .13 | .34 |
| benzene | 40.75 | 36.58 | 35.34 |
| propane | 48.35 | 49.72 | 46.14 |
| toluene | 4.43 | 6.24 | 8.55 |
| $C_8$ A | 2.87 | 2.95 | 3.52 |
| $C_9$ A | 1.48 | 1.37 | 1.44 |
| $C_{10}$ mononuclear A | 0.29 | .32 | .42 |
| $C_{11}$-$C_{12}$ mononuclear A | .02 | .03 | .02 |
| Naphthalene | .34 | .59 | .94 |
| M-Naphthalenes | .54 | .79 | 1.27 |

| Total Wt.% Conversion | 10.91 | 13.7 | 18.5 |
|---|---|---|---|
| Wt.% $C_3H_8$ Conversion | 7.7 | 9.17 | 10.74 |
| Wt.% $C_6H_6$ Conversion | 14.12 | 18.86 | 26.62 |

| Wt.% Selectivity | | | |
|---|---|---|---|
| $C_1$-$C_2$ | 0 | 0 | 2.65 |
| $C_4$ | 6.23 | 7.81 | 17.02 |
| $C_5$-$C_6$ | 1.19 | 1.31 | 1.13 |
| $C_{7+}$ A | 92.08 | 90.66 | 89.09 |

## EXAMPLE 6

A mixture of benzene and ethane in a weight ratio of about 1 :1 was fed continuously to a fixed bed isothermal tubular catalytic reactor. The catalyst is H-ZSM-5 with silica-to-alumina ratio of 40, and alpha value of 500.

The WHSV is 2.9-3.3. No $H_2$ was added.

### Table 6

| Charge (wt.%) | | |
|---|---|---|
| Ethane | 47.93 | 49.94 |
| Benzene | 52.07 | 50.06 |
| Time on Stream (hrs.) | 52.8 | 4.8 |
| Temperature (°C) | 454 | 454 |
| Pressure (Kpa) | 5497 | 5497 |
| | | |
| Product, wt % | | |
| $C_1$ | 0 | 0 |
| $C_2$ | 44.89 | 45.24 |
| propane | 0 | 0 |
| Iso-$C_4$ | 0 | 0 |
| N-$C_4$ | 0 | 0 |
| Iso-$C_5$ | 0 | 0 |
| N-$C_5$ | 0 | 0 |
| 2-M-$C_5$ | 0 | 0 |
| 3-M-$C_5$ | 0 | 0 |
| N-$C_6$ | 0 | 0 |
| $C_{13+}$ | .01 | .03 |
| benzene | 47.76 | 48.99 |
| toluene | 5.27 | 3.82 |
| $C_8$ A | 1.01 | .95 |
| $C_9$ A | .07 | .05 |
| $C_{10}$ mononuclear A | .02 | .01 |
| $C_{11}$-$C_{12}$ mononuclear A | 0 | 0 |
| Naphthalene | .57 | .49 |
| M-Naphthalenes | .41 | .4 |
| | | |
| Total Wt.% Conversion | 7.36 | 5.76 |
| Wt.% $C_2H_6$ Conversion | 6.34 | 9.41 |
| Wt.% $C_6H_6$ Conversion | 8.28 | 2.14 |

## EXAMPLE 7

A mixture of benzene and n-butane in a weight ratio of about 1 :1 was fed continuously to a fixed bed isothermal tubular catalytic reactor. The catalyst was H-ZSM-5 with silica-to-alumina ratio of 40 and alpha value of 500.

The reaction conditions are given in Table 7. The WHSV is 2.9-3.1. No $H_2$ was added.

Table 7

| Charge (wt.%) | | | | | |
|---|---|---|---|---|---|
| n-butane | 49.11 | 50.8 | 50.9 | 53 | 51 |
| Benzene | 50.9 | 49.2 | 49.1 | 47 | 49 |
| Time on Stream (hrs.) | 4.8 | 28.8 | 52.8 | 73.5 | 97.5 |
| Temperature (°C) | 371 | 343 | 316 | 399 | 398 |
| Pressure (Kpa) | 5497 | 5497 | 5497 | 5497 | 1374 |
| | | | | | |
| Product, wt % | | | | | |
| $C_1$ | .88 | .3 | .06 | 1.55 | .46 |
| $C_2$ | 2.26 | .73 | .38 | 4.76 | 1.61 |
| propane | 25.88 | 27.43 | 2.88 | 24.9 | 30.8 |
| Iso-$C_4$ | 2.87 | 4.86 | 3.52 | 2.37 | 3.07 |
| N-$C_4$ | 3.08 | 6.07 | 40.23 | 2.92 | 3.2 |
| Iso-$C_5$ | .66 | 1.49 | .59 | .59 | .61 |
| N-$C_5$ | .32 | .74 | 1.05 | .65 | .26 |
| 2-M-$C_5$ | .06 | .16 | .08 | .05 | .05 |
| 3-M-$C_5$ | .04 | .11 | .04 | .04 | .03 |
| N-$C_6$ | .03 | .09 | .12 | .04 | .03 |
| $C_{13+}$ | .25 | .26 | .01 | .32 | .19 |
| benzene | 19.16 | 28.09 | 46.37 | 8.57 | 20.85 |
| toluene | 18.55 | 9.1 | .27 | 19.17 | 17.19 |
| $C_8$ A | 13.84 | 9.49 | 1.68 | 17.3 | 11.77 |
| $C_9$ A | 5.89 | 5.63 | 1.69 | 7.63 | 4.25 |
| $C_{10}$ mononuclear A | 3.03 | 3.35 | .9 | 4.2 | 2.65 |
| $C_{11}$-$C_{12}$ mononuclear A | .53 | .83 | .08 | .84 | .57 |
| Naphthalene | .32 | .1 | 0 | .54 | .42 |
| *M-Naphthalenes | 2.28 | 1.02 | .04 | 3.47 | 1.92 |
| Total Wt.% Conversion | 77.75 | 65.84 | 13.4 | 88.51 | 75.92 |
| Wt.% N-$C_4$ Conversion | 93.73 | 88.05 | 21.02 | 94.49 | 93.6 |
| Wt.% $C_6H_6$ Conversion | 62.35 | 42.92 | 5.48 | 81.78 | 57.88 |
| | | | | | |
| Wt.% Selectivity | | | | | |
| $C_1$-$C_2$ | 4.04 | 1.56 | 3.28 | 7.13 | 2.73 |
| $C_3$ | 33.29 | 41.66 | 21.49 | 28.13 | 40.56 |
| Iso-$C_4$+$C_5$+$C_6$ | 5.16 | 11.42 | 40.3 | 4.27 | 5.39 |
| $C_7$+ Aromatics | 57.48 | 45.23 | 34.85 | 60.41 | 51.32 |

*Approximate Analysis

EXAMPLE 8

A mixture of toluene and propane in a weight ratio of about 5 :1 to 2 :1 was fed continuously to a fixed bed isothermal tubular catalytic reactor containing H-ZSM-5 with silica-to-alumina ratio equal to 40 and alpha value of 500.

The reaction conditions are given in Table 8. The WHSV is 2.9-3.6. No $H_2$ was added.

Table 8

| Charge (wt.%) | | | | | | | | | |
|---|---|---|---|---|---|---|---|---|---|
| Propane | 50.6 | 51.1 | 54.1 | 50 | 51 | 46 | 50 | 67.2 | 35.7 |
| Toluene | 49.3 | 48.8 | 45.7 | 50 | 49 | 52 | 50 | 32.6 | 64.3 |
| Time on Stream (hrs.) | 4.7 | 28.8 | 52.8 | 173 | 120 | 215 | 219 | 239 | 263 |
| Temperature (°C) | 399 | 371 | 426 | 426 | 426 | 426 | 440 | 440 | 440 |
| Pressure (Kpa) | 5497 | 5497 | 5497 | 5497 | 5497 | 5497 | 5497 | 5497 | 5497 |

Products wt %

| | | | | | | | | | |
|---|---|---|---|---|---|---|---|---|---|
| $C_1$ | 2.53 | .89 | 4.2 | 2.76 | 2.53 | 2.45 | 3.24 | 3.28 | 2.03 |
| $C_2$ | 5.37 | 1.09 | 8.79 | 5.89 | 5.1 | 4.8 | 7.1 | 7.1 | 3.8 |
| Iso-$C_4$ | 2.48 | 1.04 | 2.18 | 2.61 | 2.46 | 2.39 | 2.25 | 3.35 | 1.38 |
| N-$C_4$ | 3.21 | 1.57 | 2.89 | 3.59 | 3.32 | 3.25 | 3.06 | 4.75 | 1.92 |
| Iso-$C_5$ | .61 | .09 | .54 | .7 | .61 | .61 | .57 | .91 | .31 |
| N-$C_5$ | .34 | .08 | .34 | .45 | .38 | .4 | .35 | .61 | .19 |
| 2-M-$C_5$ | .05 | .02 | .05 | .07 | .06 | .06 | .06 | .1 | .03 |
| 3-M-$C_5$ | .03 | .01 | .03 | .04 | .04 | .04 | .04 | .06 | .02 |
| N-$C_6$ | .07 | .02 | .04 | .05 | .05 | .05 | .05 | .08 | .02 |
| $C_{13+}$ | .08 | .01 | .11 | .02 | .01 | .01 | .02 | 0 | .01 |
| benzene | 4.84 | 7.83 | ·3.98 | 4.48 | 5.15 | 5.19 | 4.76 | 2.99 | 8.44 |
| propane | 29.36 | 42.69 | 24.95 | 30.53 | 29.71 | 29.96 | 26.54 | 39.02 | 18.88 |
| toluene | 17.07 | 21.37 | 15.42 | 16.2 | 17.45 | 17.85 | 17.03 | 11.88 | 24.75 |
| $C_8$ A | 18.4 | 15.52 | 18.21 | 18.18 | 18.97 | 19.12 | 19.17 | 14.08 | 23.57 |
| $C_9$ A | 8.57 | 5.32 | 8.64 | 8.11 | 8.2 | 8.16 | 8.33 | 6.38 | 8.82 |
| $C_{10}$ mononuclear A | 3.93 | 1.91 | 4.31 | 3.56 | 3.47 | 3.39 | 3.57 | 2.74 | 3.29 |
| $C_{11}$-$C_{12}$ mononuclear A | .45 | .11 | .87 | .37 | .53 | .49 | .60 | .30 | .40 |
| Naphthalene | .22 | .04 | .5 | .3 | .28 | .25 | 5 | .33 | .35 |
| *M-Naphthalenes | 2.3 | .37 | 3.84 | 1.98 | 1.59 | 1.42 | 2.65 | 1.85 | 1.77 |

| | | | | | | | | | |
|---|---|---|---|---|---|---|---|---|---|
| Total Wt.% Conversion | 53.57 | 35.94 | 59.63 | 53.27 | 52.85 | 52.19 | 56.43 | 49.1 | 56.35 |
| Wt.% $C_3H_8$ Conversion | 41.95 | 16.39 | 53.88 | 38.89 | 41.81 | 37.05 | 46.88 | 41.9 | 47.04 |
| Wt.% Toluene Conversion | 65.37 | 56.19 | 66.28 | 67.52 | 64.23 | 65.85 | 65.86 | 63.59 | 61.48 |

Wt.% Selectivity

| | | | | | | | | | |
|---|---|---|---|---|---|---|---|---|---|
| $C_1$-$C_2$ | 14.75 | 5.51 | 21.78 | 16.24 | 14.44 | 13.89 | 18.29 | 21.12 | 10.27 |
| $C_4$ | 10.62 | 7.26 | 8.5 | 11.64 | 10.94 | 10.81 | 9.41 | 16.5 | 5.85 |
| $C_5$-$C_6$ | 2.1 | .61 | 1.78 | 2.57 | 2.27 | 2.32 | 2 | 3.83 | 1.05 |
| $C_{6+}$ A | 72.41 | 86.56 | 67.85 | 69.46 | 72.28 | 72.87 | 70.18 | 58.39 | 82.74 |

*Approximate Analysis

## EXAMPLE 9

A mixture of benzene and propane in a weight ratio of about 2 :1 to 5 :1 was fed continuously to a fixed bed isothermal tubular catalytic reactor containing H-ZSM-5 with silica-to-alumina ratio of 40 and alpha value of 500.

The reaction conditions are given in Table 9. The WHSV is 2.8-3.0. No $H_2$ was added.

### Table 9

| Charge (wt.%) | | | | |
|---|---|---|---|---|
| Propane | 34.3 | 51.2 | 65.8 | 49.1 |
| Benzene | 65.6 | 48.6 | 34.1 | 49.1 |
| Time on Stream (hrs.) | 5.2 | 5.3 | 5.2 | 5.2 |
| Temperature (°C) | 455 | 453 | 455 | 426 |
| Pressure (Kpa) | 5497 | 5497 | 5497 | 5497 |

| Product, wt % | | | | |
|---|---|---|---|---|
| $C_1$ | 7.23 | 7.9 | 9.29 | 5.73 |
| $C_2$ | 9.99 | 13.23 | 18.47 | 9.42 |
| Iso-$C_4$ | .25 | .66 | 1.48 | 1.21 |
| N-$C_4$ | .33 | .89 | 1.94 | 1.62 |
| Iso-$C_5$ | .03 | .08 | .18 | .17 |
| N-$C_5$ | .02 | .06 | .14 | .12 |
| 2-M-$C_5$ | 0 | .01 | .03 | 0 |
| 3-M-$C_5$ | 0 | .01 | .02 | .02 |
| N-$C_6$ | 0 | .01 | .02 | .01 |
| *$C_{13+}$ | .44 | .46 | .35 | .22 |
| benzene | 14.21 | 9.25 | 4.31 | 9.99 |
| propane | 4.32 | 9.92 | 20.27 | 16.41 |
| toluene | 27.84 | 22 | 12.87 | 22.33 |
| $C_8$ A | 19.13 | 17.82 | 12.84 | 18.29 |
| $C_9$ A | 5.52 | 6.17 | 5.43 | 6.73 |
| $C_{10}$ mononuclear A | 2.32 | 2.93 | 2.99 | 3.25 |
| $C_{11}$-$C_{12}$ mononuclear A | .16 | .58 | .77 | .53 |
| Naphthalene | 1.65 | 1.32 | 1.2 | .59 |
| *M-Naphthalenes | 6.56 | 6.68 | 7.33 | 3.3 |

| | | | | |
|---|---|---|---|---|
| Total Wt.% Conversion | 81.47 | 80.83 | 75.43 | 73.59 |
| Wt.% $C_3H_8$ Conversion | 87.41 | 80.63 | 69.17 | 66.55 |
| Wt.% $C_6H_6$ Conversion | 78.34 | 80.98 | 87.35 | 80.33 |
| Wt.% Selectivity | | | | |
| $C_1$-$C_2$ | 21.14 | 26.14 | 36.8 | 20.59 |
| $C_4$ | .71 | 1.92 | 4.55 | 3.85 |
| $C_5$-$C_6$ | .06 | .21 | .56 | .48 |
| $C_{7+}$ A | 78.09 | 71.72 | 58.04 | 75.06 |

*Approximate Analysis

## EXAMPLE 10

A mixture of benzene and propane in a weight ratio of about 5 :1 to 2 :1 was fed continuously to a fixed bed isothermal tubular catalytic reactor containing H-ZSM-5 with silica-to-alumina ratio of 40 and alpha of 500.

The WHSV is 2.9-3.1. No $H_2$ was added.

EP 0 250 879 B1

Table 10

| Charge (wt.%) | | | | |
|---|---|---|---|---|
| Propane | 49.1 | 50.8 | 33.9 | 67.5 |
| Benzene | 50.8 | 49.1 | 66 | 32.3 |
| Time on Stream (hrs.) | 5.2 | 4.8 | 5.3 | 5.2 |
| Temperature (°C) | 426 | 399 | 397 | 399 |
| Pressure (Kpa) | 5497 | 5497 | 5497 | 5497 |

Product, wt %

| | | | | |
|---|---|---|---|---|
| $C_1$ | 5.73 | 2.06 | 1.23 | 2.40 |
| $C_2$ | 9.42 | 2.15 | .87 | 3.24 |
| Iso-$C_4$ | 1.21 | 1.64 | .44 | 2.9 |
| N-$C_4$ | 1.62 | 2.22 | .65 | 3.78 |
| Iso-$C_5$ | .17 | .18 | .04 | .51 |
| N-$C_5$ | .12 | .15 | 0 | .34 |
| 2-M-$C_5$ | 0 | .02 | .01 | .05 |
| 3-M-$C_5$ | .02 | .01 | 0 | .03 |
| N-$C_6$ | .01 | .02 | 0 | .04 |
| *$C_{13+}$ | .22 | 0 | .01 | .1 |
| benzene | 9.99 | 30.65 | 51.53 | 17.6 |
| propane | 16.41 | 34.99 | 24.53 | 46.13 |
| toluene | 22.33 | 10.62 | 6.23 | 10.2 |
| $C_8$ A | 18.29 | 10.32 | 10.99 | 7.88 |
| $C_9$ A | 6.73 | 2.93 | 1.99 | 2.84 |
| $C_{10}$ mononuclear A | 3.25 | 1.35 | .81 | 1.37 |
| $C_{11}$-$C_{12}$ mononuclear A | .53 | .14 | .04 | .18 |
| Naphthalene | .59 | .11 | .14 | .08 |
| *M-Naphthalenes | 3.3 | .41 | .44 | .28 |

| | | | | |
|---|---|---|---|---|
| Total Wt.% Conversion | 73.59 | 34.36 | 23.94 | 36.27 |
| Wt.% $C_3H_8$ Conversion | 66.55 | 32.13 | 27.7 | 31.65 |
| Wt.% $C_6H_6$ Conversion | 80.33 | 37.55 | 21.89 | 45.53 |

Wt.% Selectivity

| | | | | |
|---|---|---|---|---|
| $C_1$-$C_2$ | 20.59 | 12.25 | 8.77 | 15.55 |
| $C_4$ | 3.85 | 11.23 | 4.55 | 18.42 |
| $C_5$-$C_6$ | .48 | 1.11 | .38 | 2.76 |
| $C_{7+}$ A | 75.06 | 75.32 | 86.26 | 63.22 |

EXAMPLE 11

A mixture of benzene and propane was fed continuously to a fixed bed isothermal tubular catalytic reactor containing H-ZSM-5 with silica-to-alumina ratio of 40 and alpha value of 500.

The reaction conditions are given in Table 11. The WHSV is 1.7-3.2. No $H_2$ was added.

13

## Table 11

| Charge (wt.%) | | | | | |
|---|---|---|---|---|---|
| Propane | 50.2 | 50.4 | 52.4 | 52.7 | 53.5 |
| Benzene | 49.6 | 49.5 | 47.5 | 47.2 | 46.3 |
| Time on Stream (hrs.) | 5.4 | 28.9 | 52.9 | 76.9 | 101.2 |
| Temperature (°C) | 426 | 426 | 440 | 454 | 440 |
| Pressure (Kpa) | 2748 | 687 | 206 | 137 | 137 |

| Product, wt % | | | | | |
|---|---|---|---|---|---|
| $C_1$ | 3.51 | 1.77 | 1.52 | 2.6 | 2.7 |
| $C_2$ | 6.74 | 2.45 | 1.92 | 3.69 | 4.52 |
| Iso-$C_4$ | 1.69 | 1.27 | 1.18 | 1.23 | 1.36 |
| N-$C_4$ | 2.2 | 1.86 | 1.72 | 1.64 | 1.78 |
| Iso-$C_5$ | .23 | .12 | .1 | .09 | .12 |
| N-$C_5$ | .16 | .1 | .09 | .09 | .1 |
| 2-M-$C_5$ | .02 | .01 | .01 | .01 | .01 |
| 3-M-$C_5$ | .01 | .01 | 0 | 0 | 0 |
| N-$C_6$ | .02 | .01 | .01 | .01 | .01 |
| *$C_{13+}$ | .07 | 0 | 0 | .0 | .01 |
| benzene | 14.16 | 34.18 | 38.54 | 35.83 | 36.89 |
| propane | 24.63 | 36.37 | 38.7 | 35.5 | 36.8 |
| toluene | 21.9 | 11.61 | 8.27 | 9.81 | 7.59 |
| $C_8$ A | 14.37 | 7.05 | 5.53 | 5.83 | 4.86 |
| $C_9$ A | 4.95 | 1.72 | 1.09 | 1.23 | 1.09 |
| $C_{10}$ mononuclear A | 2.35 | .78 | .59 | .71 | .54 |
| $C_{11}$-$C_{12}$ mononuclear A | .37 | .1 | .02 | .03 | .02 |
| Naphthalene | .43 | .13 | .12 | .27 | .28 |
| *M-Naphthalenes | 2.15 | .43 | .32 | .82 | .96 |

| Total Wt.% Conversion | 61.21 | 29.45 | 22.6 | 28.5 | 26.2 |
|---|---|---|---|---|---|
| Wt.% $C_3H_8$ Conversion | 50.96 | 27.79 | 26.09 | 32.59 | 31.2 |
| Wt.% $C_6H_6$ Conversion | 71.46 | 30.91 | 18.81 | 24.04 | 20.39 |
| Wt.% Selectivity | | | | | |
| $C_1$-$C_2$ | 16.75 | 14.33 | 15.4 | 22.74 | 27.88 |
| $C_4$ | 6.34 | 10.66 | 12.88 | 10.32 | 12.28 |
| $C_5$-$C_6$ | .75 | .95 | 1.11 | .84 | 1.07 |
| $C_{7+}$ A | 76.12 | 74.09 | 70.53 | 65.65 | 58.7 |

## EXAMPLE 12

A mixture of benzene and propane was fed continuously to a fixed bed isothermal tubular catalytic reactor containing H-ZSM-5 with silica-to-alumina ratio of 40 and alpha of 500.

The WHSV of the feed is 3-3.1. $H_2$ was added some of the time.

Results in Table 12 show the alkylation selectivity as measured by the $C_7+$ aromatics decreases when $H_2$ is added due to increased hydrocracking.

## Table 12

| Charge (wt.%) | | |
|---|---|---|
| Propane | 51.2 | 50.7 |
| Benzene | 48.6 | 49.1 |
| $H_2$/Hydrocarbon mole ratio | 0/1 | .4/1 |
| Time on Stream (hrs.) | 5.3 | 5.2 |
| Temperature (°C) | 453 | 456 |
| Pressure (Kpa) | 5497 | 5497 |
| | | |
| **Product, wt %** | | |
| $C_1$ | 7.9 | 10.73 |
| $C_2$ | 13.23 | 14.79 |
| Iso-$C_4$ | .66 | .68 |
| N-$C_4$ | .89 | .84 |
| Iso-$C_5$ | .08 | .09 |
| N-$C_5$ | .06 | .05 |
| 2-M-$C_5$ | .01 | .01 |
| 3-M-$C_5$ | .01 | .01 |
| N-$C_6$ | .01 | 0 |
| *$C_{13+}$ | .46 | .05 |
| benzene | 9.25 | 16.08 |
| propane | 9.92 | 12.46 |
| toluene | 22 | 23.32 |
| $C_8$ A | 17.82 | 13.64 |
| $C_9$ A | 6.17 | 3.95 |
| $C_{10}$ mononuclear A | 2.93 | 1.47 |
| $C_{11}$-$C_{12}$ mononuclear A | .58 | .1 |
| Naphthalene | 1.32 | .4 |
| *M-Naphthalenes | 6.68 | 1.33 |
| **Total Wt.% Conversion** | 80.83 | 71.46 |
| **Wt.% $C_3H_8$ Conversion** | 80.63 | 75.45 |
| **Wt.% $C_6H_6$ Conversion** | 80.98 | 67.24 |
| **Wt.% Selectivity** | | |
| $C_1$-$C_2$ | 26.14 | 35.71 |
| $C_4$ | 1.92 | 2.13 |
| $C_5$-$C_6$ | .21 | .22 |
| $C_{7+}$ A | 71.22 | 61.94 |

*Approximate Analysis

## EXAMPLE 13

A mixture of benzene and propane was fed continuously to a fixed bed isothermal tubular catalytic reactor containing gallium-substituted ZSM-5 with silica-to-metal oxide ($Ga_2O_3$ + $Al_2O_3$) ratio of 42 and alpha value of 500.

The reaction conditions are given in Table 13. The WHSV of the feed is 2.8-3.1. No $H_2$ was added.

## Table 13

| Charge (wt.%) | | |
|---|---|---|
| Propane | 38.1 | 36.8 |
| Benzene | 61.8 | 63.1 |
| Time on Stream (hrs.) | 30.4 | 54.4 |
| Temperature (°C) | 399 | 454 |
| Pressure (Kpa) | 5497 | 5497 |
| Catalyst Type: [Ga] ZSM-5 | | |

| Product, wt % | | |
|---|---|---|
| $C_1$ | .41 | 4.15 |
| $C_2$ | .1 | 3.47 |
| Iso-$C_4$ | .08 | .93 |
| N-$C_4$ | .18 | 1.24 |
| Iso-$C_5$ | .01 | .12 |
| N-$C_5$ | .01 | .09 |
| 2-M-$C_5$ | 0 | .01 |
| 3-M-$C_5$ | 0 | .01 |
| N-$C_6$ | .01 | .01 |
| *$C_{13+}$ | .25 | .12 |
| benzene | 58.04 | 29.19 |
| propane | 33.4 | 15.12 |
| toluene | 1.45 | 21.68 |
| $C_8$ A | 3.48 | 12.04 |
| $C_9$ A | 2.14 | 5.33 |
| $C_{10}$ mononuclear A | .23 | 1.94 |
| $C_{11}$-$C_{12}$ mononuclear A | .2 | .23 |
| Naphthalene | 0 | 1.23 |
| *M-Naphthalenes | 0 | 3 |
| **Total Wt.% Conversion** | 8.56 | 55.69 |
| **Wt.% $C_3H_8$ Conversion** | 14.01 | 58.92 |
| **Wt.% $C_6H_6$ Conversion** | 14.01 | 53.73 |
| **Wt.% Selectivity** | | |
| $C_1$-$C_2$ | 5.96 | 13.68 |
| $C_4$ | 3.04 | 3.9 |
| $C_5$-$C_6$ | .35 | .43 |
| $C_{7+}$ A | 90.54 | 81.99 |

## EXAMPLE 14

Benzene and propane in a weight ratio of about 1:1 was fed to a fixed bed isothermal tubular catalytic reactor containing a zinc-substituted ZSM-5 with silica-to-alumina ratio of 70.

The reaction conditions are given in Table 14. The WHSV of the feed is 1.3-2.9. No $H_2$ was added.

16

## Table 14

| Charge (wt.%) | | |
|---|---|---|
| Propane | 47.7 | 50.9 |
| Benzene | 52.2 | 49 |
| Time on Stream (hrs.) | 4.9 | 25.2 |
| Temperature (°C) | 468 | 468 |
| Pressure (Kpa) | 137 | 137 |
| | | |
| **Product, wt %** | | |
| $C_1$ | 1.13 | 2.55 |
| $C_2$ | .78 | 1.91 |
| Iso-$C_4$ | .28 | .32 |
| N-$C_4$ | .65 | .76 |
| Iso-$C_5$ | .02 | .03 |
| N-$C_5$ | .03 | .04 |
| 2-M-$C_5$ | 0 | 0 |
| 3-M-$C_5$ | 0 | 0 |
| N-$C_6$ | 0 | 0 |
| *$C_{13+}$ | 0 | 0 |
| benzene | 51.11 | 44.78 |
| propane | 39.16 | 36.21 |
| toluene | 3 | 7.08 |
| $C_8$ A | 2.23 | 3.47 |
| $C_9$ A | .43 | .66 |
| $C_{10}$ mononuclear A | .19 | .32 |
| $C_{11}$-$C_{12}$ mononuclear A | .01 | .02 |
| Naphthalene | .29 | .7 |
| *M-Naphthalenes | .31 | .72 |
| **Total Wt.% Conversion** | 9.57 | 18.85 |
| **Wt.% $C_3H_8$ Conversion** | 17.87 | 28.85 |
| **Wt.% $C_6H_6$ Conversion** | 2.01 | 8.52 |
| **Wt.% Selectivity** | | |
| $C_1$-$C_2$ | 22.32 | 24.4 |
| $C_4$ | 9.82 | 5.99 |
| $C_5$-$C_6$ | .63 | .48 |
| $C_{7+}$ A | 67.5 | 68.8 |

*Approximate Analysis

## EXAMPLE 15

Benzene and propane in a weight ratio of about 1 :1 was fed continuously to a fixed bed isothermal tubular catalytic reactor containing a zinc and rhenium exchanged H-ZSM-5 with silica-to-alumina ratio of 35.

The reaction conditions are given in Table 15. The WHSV of the feed is 3.3. No $H_2$ was added.

## Table 15

| Charge (wt.%) | |
|---|---|
| Propane | 54.1 |
| Benzene | 45.8 |
| Time on Stream (hrs.) | 4.9 |
| Temperature (°C) | 468 |
| Pressure (Kpa) | 137 |

| Product, wt % | |
|---|---|
| $C_1$ | .42 |
| $C_2$ | 1.91 |
| Iso-$C_4$ | .42 |
| N-$C_4$ | .24 |
| Iso-$C_5$ | .02 |
| N-$C_5$ | .01 |
| 2-M-$C_5$ | 0 |
| 3-M-$C_5$ | 0 |
| N-$C_6$ | 0 |
| *$C_{13+}$ | 0 |
| benzene | 47.28 |
| propane | 44.83 |
| toluene | 1.17 |
| $C_8$ A | 1.27 |
| $C_9$ A | 1 |
| $C_{10}$ mononuclear A | .26 |
| $C_{11}$-$C_{12}$ mononuclear A | .02 |
| Naphthalene | .18 |
| *M-Naphthalenes | .2 |
| Total Wt.% Conversion | 9.22 |
| Wt.% $C_3H_8$ Conversion | 9.22 |
| Wt.% $C_6H_6$ Conversion | 0 |
| Wt.% Selectivity | |
| $C_1$-$C_2$ | 25.27 |
| $C_4$ | 7.16 |
| $C_5$-$C_6$ | .54 |
| $C_{7+}$ A | 44.47 |

*Approximate Analysis

## EXAMPLE 16

Benzene and propane in a weight ratio of about 1:1 was fed continuously to a fixed bed isothermal tubular catalytic reactor containing a 0.38 wt % Pt on H-ZSM-5 with silica-to-alumina ratio of 70.

The reaction conditions are given in Table 16. The WHSV is 1.3-1.5. No $H_2$ is added.

## Table 16

| Charge (wt.%) | | |
|---|---|---|
| Propane | | |
| Benzene | | |
| Time on Stream (hrs.) | 4.8 | 24.8 |
| Temperature (°C) | 426 | 440 |
| Pressure (Kpa) | 5497 | 137 |
| | | |
| Product, wt % | | |
| $C_1$ | 2.94 | .71 |
| $C_2$ | 16.32 | 2.47 |
| Iso-$C_4$ | 1.36 | 1.12 |
| N-$C_4$ | 1.58 | 1.66 |
| Iso-$C_5$ | .20 | .12 |
| N-$C_5$ | .13 | .12 |
| 2-M-$C_5$ | .02 | .01 |
| 3-M-$C_5$ | .01 | .01 |
| N-$C_6$ | .02 | .01 |
| *$C_{13+}$ | .21 | 0 |
| benzene | 18.66 | 38.67 |
| propane | 16.56 | 45.84 |
| toluene | 21.57 | 4.1 |
| $C_8$ A | 10.87 | 2.73 |
| $C_9$ A | 3.57 | .86 |
| $C_{10}$ mononuclear A | 1.99 | .44 |
| $C_{11}$-$C_{12}$ mononuclear A | .1 | .02 |
| Naphthalene | 1.07 | .19 |
| *M-Naphthalenes | 2.79 | .33 |
| Total Wt.% Conversion | 64.79 | 51.32 |
| Wt.% $C_3H_8$ Conversion | 64.47 | 15.19 |
| Wt.% $C_6H_6$ Conversion | 64.96 | 15.53 |
| Wt.% Selectivity | | |
| $C_1$-$C_2$ | 29.73 | 20.76 |
| $C_4$ | 4.54 | 18.15 |
| $C_5$-$C_6$ | .62 | 2.35 |
| $C_{7+}$ A | 65.09 | 56.59 |

*Approximate Analysis

## EXAMPLE 17

Benzene and propane in a weight ratio of about 1 :1 was fed continuously to a fixed bed isothermal tubular catalytic reactor containing a 4 wt % Ni exchanged (dried without washing off excess nickel) H-ZSM-5 with silica-to-alumina ratio of 70.

The reaction conditions are given in Table 17. The WHSV is 1.2-1.3. No $H_2$ is added.

<u>Table 17</u>

| Charge (wt.%) | | |
|---|---|---|
| Propane | 54.4 | 49.3 |
| Benzene | 45.5 | 50.5 |
| Time on Stream (hrs.) | 5.3 | 28.8 |
| Temperature (°C) | 426 | 440 |
| Pressure (Kpa) | 5497 | 137 |
| | | |
| <u>Product, wt %</u> | | |
| $C_1$ | 4.58 | 1.06 |
| $C_2$ | 4.98 | 1.06 |
| Iso-$C_4$ | 1.86 | .28 |
| N-$C_4$ | 2.57 | .52 |
| Iso-$C_5$ | .26 | .02 |
| N-$C_5$ | .2 | .03 |
| 2-M-$C_5$ | .03 | 0 |
| 3-M-$C_5$ | .02 | 0 |
| N-$C_6$ | .03 | 0 |
| *$C_{13+}$ | .08 | 0 |
| benzene | 19.32 | 48.48 |
| propane | 27.97 | 41.4 |
| toluene | 17.36 | 3.61 |
| $C_8$ A | 11.18 | 1.9 |
| $C_9$ A | 4.37 | .42 |
| $C_{10}$ mononuclear A | 2.21 | .22 |
| $C_{11}$-$C_{12}$ mononuclear A | .2 | .02 |
| Naphthalene | .7 | .35 |
| M-Naphthalenes | 2.04 | .46 |
| <u>Total Wt.% Conversion</u> | 52.71 | 9.97 |
| <u>Wt.% $C_3H_8$ Conversion</u> | 48.55 | 16.09 |
| <u>Wt.% $C_6H_6$ Conversion</u> | 57.51 | 4.02 |
| | | |
| <u>Wt.% Selectivity</u> | | |
| $C_1$-$C_2$ | 18.14 | 21.26 |
| $C_4$ | 8.4 | 8.12 |
| $C_5$-$C_6$ | 1.06 | .8 |
| $C_{7+}$ A | 72.36 | 70.01 |

*Approximate Analysis

## EXAMPLE 18

Benzene and propane in a weight ratio of about 1 :1 was fed continuously to a fixed bed isothermal tubular catalytic reactor containing 0.2 wt % Cr exchanged H-ZSM-5 with silica-to-alumina ratio of 70.

The reaction conditions are given in Table 18. The WHSV is 1.3-1.4. No $H_2$ is added.

## Table 18

| Charge (wt.%) | | |
|---|---|---|
| Propane | 50.2 | 51 |
| Benzene | 49.6 | 49 |
| Time on Stream (hrs.) | 5 | 25 |
| Temperature (°C) | 426 | 440 |
| Pressure (Kpa) | 5497 | 137 |
| **Product, wt %** | | |
| $C_1$ | 5.48 | 1.16 |
| $C_2$ | 7.41 | 1.38 |
| Iso-$C_4$ | 1.43 | .97 |
| N-$C_4$ | 1.81 | 1.51 |
| Iso-$C_5$ | .19 | .08 |
| N-$C_5$ | .13 | .09 |
| 2-M-$C_5$ | .02 | .01 |
| 3-M-$C_5$ | .01 | 0 |
| N-$C_6$ | .02 | .01 |
| *$C_{13+}$ | .19 | 0 |
| benzene | 14.86 | 44.33 |
| propane | 18.74 | 41.77 |
| toluene | 22.7 | 4.21 |
| $C_8$ A | 14.95 | 2.64 |
| $C_9$ A | 5.85 | .64 |
| $C_{10}$ mononuclear A | 2.34 | .35 |
| $C_{11}$-$C_{12}$ mononuclear A | .3 | .03 |
| Naphthalene | .61 | .14 |
| *M-Naphthalenes | 2.92 | .25 |
| **Total Wt.% Conversion** | 66.39 | 13.74 |
| **Wt.% $C_3H_8$ Conversion** | 62.68 | 18.06 |
| **Wt.% $C_6H_6$ Conversion** | 70.05 | 9.16 |
| **Wt.% Selectivity** | | |
| $C_1$-$C_2$ | 19.42 | 19.07 |
| $C_4$ | 4.88 | 18.41 |
| $C_5$-$C_6$ | .57 | 1.67 |
| $C_{7+}$ A | 75.1 | 60.12 |

*Approximate Analysis

Examples 1-18 illustrate the effectiveness of converting benzene or toluene to alkylated aromatic materials in the presence of ethane, propane or n-butane. The aromatics formed are predominantly $C_7$-$C_9$ materials.

In particular, the aromatics present in the product contain a substantial amount of toluene, as indicated by Tables 1-18 in the Examples. Toluene is produced during the alkylation reactions in an amount from about 30% by weight to about 75% by weight, based on total yield or aromatic hydrocarbons. A toluene rich aromatic product is obtained when a mononuclear aromatic hydrocarbon such as benzene is reacted with ethane, propane, butanes or mixtures thereof. The process of the invention can upgrade gasoline fractions by reducing benzene concentration, generating more volume of gasoline, reducing gasoline density, and improving octane quality. In the petrochemicals manufacturing industry, the process affords an economical approach to producing alkylated aromatic materials from simple starting materials, such as benzene and propane.

The process of the present invention achieves alkylation selectivities above 50%, whereas prior art process (using cracking to obtain an olefin and a paraffin fragment) were limited to an alkylation selectivity of about 50%.

EP 0 250 879 B1

**Claims**

1. A process for making alkyl aromatic hydrocarbons characterized by contacting a feed substantially free of hydrogen and comprising mononuclear aromatic compound and a $C_2$-$C_4$ alkane in a weight ratio of 10 :1 to 1 :10, at 315°C to 480°C and a pressure of 100 to 10,000 kPa with a catalyst comprising acidic medium pore metallosilicate zeolite.

2. The process of claim 1 further characterized in that the reaction mixture is substantially free of added olefin and the alkyl aromatics produced are predominantly $C_7$ to $C_9$ aromatic hydrocarbons.

3. The process of claim 2 further characterized in that the zeolite consists essentially of ZSM-5, ZSM-11, ZSM-22, ZSM-23, ZSM-35, ZSM-38, ZSM-45, ZSM-48 and/or ZSM-50.

4. The process of claim 3 further characterized in that the zeolite HZSM-5.

5. The process of any preceding claim further characterized in that the aromatic compound is benzene.

6. The process of any preceding claim further characterized in that the alkane is ethane.

7. The process of any of claims 1 to 5 further characterized in that the alkane is propane.


**Ansprüche**

1. Verfahren zur Herstellung aromatischer Alkylkohlenwasserstoffe, gekennzeichnet durch den Kontakt einer Zufuhr, die im wesentlichen frei von Wasserstoff ist und eine einkernige aromatische Verbindung und ein $C_2$-$C_4$-Alkan in einem Gewichtsverhältnis von 10 :1 bis 1 :10 umfaßt, bei 315°C bis 480°C und einem Druck von 100 bis 10.000 kPa mit einem Katalysator, der einen sauren MetallsilicatZeolith mit mittleren Poren umfaßt.

2. Verfahren nach Anspruch 1, weiterhin dadurch gekennzeichnet, daß die Reaktionsmischung im wesentlichen frei von zugegebenem Olefin ist und die erzeugten Alkylaromaten vorwiegend aromatische $C_7$-$C_9$-Kohlenwasserstoffe sind.

3. Verfahren nach Anspruch 2, weiterhin dadurch gekennzeichnet, daß der Zeolith im wesentlichen aus ZSM-5, ZSM-11, ZSM-22, ZSM-23, ZSM-35, ZSM-38, ZSM-45, ZSM-48 und/oder ZSM-50 besteht.

4. Verfahren nach Anspruch 3, weiterhin dadurch gekennzeichnet, daß der Zeolith HZSM-5 ist.

5. Verfahren nach einem der vorstehenden Ansprüche, weiterhin dadurch gekennzeichnet, daß die aromatische Verbindung Benzol ist.

6. Verfahren nach einem der vorstehenden Ansprüche, weiterhin dadurch gekennzeichnet, daß das Alkan Ethan ist.

7. Verfahren nach einem der Ansprüche 1 bis 5, weiterhin dadurch gekennzeichnet, daß das Alkan Propan ist.


**Revendications**

1. Procédé de préparation d'hydrocarbures alkyles aromatiques, caractérisé en ce qu'il consiste à mettre en contact une charge, sensiblement exempte d'hydrogène et comprenant un composé aromatique monocyclique et un alcane en $C_2$-$C_4$ dans un rapport pondéral de 10/1 à 1/10, à une température de 315-480°C et sous une pression de 100 à 10000 kPa, avec un catalyseur comprenant une zéolite du type métallosilicate, acide, à pores de diamètre moyen.

2. Le procédé selon la revendication 1, caractérisé en outre en ce que le mélange réactionnel est pratiquement exempt d'oléfines ajoutées et que les alkyles aromatiques produits sont essentiellement des hydrocarbures aromatiques en $C_7$ à $C_9$.

3. Le procédé selon la revendication 2, caractérisé en outre en ce que la zéolite est constituée essentiellement de ZSM-5, ZSM-11, ZSM-22, ZSM-23, ZSM-35, ZSM-38, ZSM-45, ZSM-48 et/ou ZSM-50.

4. Le procédé selon la revendication 3, caractérisé en outre en ce que la zéolite est la H-ZSM-5.

5. Le procédé selon l'une quelconque des revendications précédentes, caractérisé en outre en ce que le composé aromatique est le benzène.

6. Le procédé selon l'une quelconque des revendications précédentes, caractérisé en outre en ce que l'alcane est l'éthane.

7. Le procédé selon l'une quelconque des revendications 1 à 5, caractérisé en outre en ce que l'alcane est le propane.

22